(19) Europäisches Patentamt
European Patent Office
Office européen des brevets

(11) Veröffentlichungsnummer: **0 218 127 B1**

# (12) EUROPÄISCHE PATENTSCHRIFT

(45) Veröffentlichungstag der Patentschrift: **13.12.89**

(51) Int. Cl.⁴: **C12Q 1/60**

(21) Anmeldenummer: **86112875.9**

(22) Anmeldetag: **18.09.86**

## (54) Verfahren und Reagenz zur spezifischen Bestimmung von HDL-Cholesterin im Serum.

(30) Priorität: **18.09.85 DE 3533288**

(43) Veröffentlichungstag der Anmeldung: **15.04.87 Patentblatt 87/16**

(45) Bekanntmachung des Hinweises auf die Patenterteilung: **13.12.89 Patentblatt 89/50**

(84) Benannte Vertragsstaaten: **AT BE CH DE FR GB IT LI LU NL SE**

(56) Entgegenhaltungen:
**EP-A- 0 091 026**
**US-A- 4 105 521**
**US-A- 4 275 152**
**US-A- 4 414 326**

**CLINICAL CHEMISTRY, Band 26, Nr. 13, 1980, Seiten 1780-1786, Easton, Pennsylvania, US; P.N.M. DEMACKER et al.: "Measurement of high-density lipoprotein cholesterol in serum: Comparison of six isolation methods combined with enzymic cholesterol analysis" C et IRTF" D. NAUK UZB. SSR, FIZ.-TEKH. INST. 1966, 65-77 000**
**PATENT ABSTRACTS OF JAPAN, Band 7, Nr. 7 (C-144)[1152], 12. Januar 1983; & JP - A - 57 163 500 (SHINOTESUTO KENKYUSHO K.K.) 07.10.1982**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof(DE)**

(72) Erfinder: **Kerscher, Lorenz, Dr. rer. nat., Paul-Loebe-Strasse 21, D-8122 Penzberg(DE)**
Erfinder: **Siedel, Joachim, Dr. rer. nat., Bahnhofstrasse 6, D-8131 Bernried(DE)**
Erfinder: **Ziegenhorn, Joachim, Dr. rer. nat., Ina-Seidel-Weg 1, D-8130 Starnberg(DE)**
Erfinder: **Pautz, Brigitte, Kientalstrasse 27, D-8036 Herrsching(DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86(DE)**

EP 0 218 127 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Neben der im klinischen Labor seit langem fest etablierten Bestimmung des Gesamtcholesterins im Serum (oder Plasma) hat in den letzten Jahren die zusätzliche Analyse des an die "high density lipoprotein" - Fraktion gebundenen Cholesterins (HDL-Cholesterin) zur verbesserten Diagnose des Atherosklerose- bzw. Herzinfarktrisikos eine erhebliche Bedeutung erlangt.

Umfangreiche klinische Studien haben erwiesen, daß ein erhöhter Serum-Gesamtcholesterinspiegel (Summe des an die Lipoproteinfraktionen Chylomikronen, VLDL ("very low density lipoproteins"), LDL ("low density lipoproteins") sowie HDL gebundenen freien und fettsäureveresterten Cholesterins) zu einem gesteigerten Herzinfarktrisiko führt, während andererseits eine inverse Beziehung zwischen dem Serum-HDL-Cholesterin-Gehalt und der Gefahr atherosklerotischer Blutgefäßveränderungen besteht (s. z. B. G. Assmann, Lipidstoffwechsel und Atherosklerose, Schattauer-Verlag, Stuttgart 1982).

Für die Bestimmung des Serum-Gesamtcholesterins stehen seit etwa 15 Jahren einfache, aber dennoch sehr genaue vollenzymatische Analysenverfahren zur Verfügung, die sowohl manuell als auch gleichermaßen gut an automatisierten Analysensystemen ohne jeglichen Probenvorbehandlungsschritt durchführbar sind und üblicherweise nach dem Reaktionsschema

$$1)\quad \text{Cholesterinester} + H_2O \xrightarrow[\text{Esterase}]{\text{Cholesterin-}} \text{Cholesterin} + \text{Fettsäure}$$

$$2)\quad \text{Cholesterin} + O_2 \xrightarrow[\text{Oxidase}]{\text{Cholesterin-}} \Delta^4\text{-Cholesten-3-on} + H_2O_2$$

Der Cholesterinnachweis erfolgt entweder über das gebildete $H_2O_2$, bevorzugt colorimetrisch, insbesondere durch die Peroxidase-katalysierte oxidative Kupplung von Phenol, Phenol- oder Anilinderivaten mit 4-Aminoantipyrin zu einem roten bis violetten Chinoniminfarbstoff ("Trinder-Reaktion"), oder über das gebildete Cholestenon oder über das in Gleichung 2) verbrauchte $O_2$. Diese Bestimmungsmethoden sind dem Fachmann bekannt, z. B. aus den DE-OS 22 24 132 und 25 06 712.

Ein wesentliches Merkmal des entsprechenden Reaktionssystems bzw. Analysenreagenzes ist die Anwesenheit von Detergenzien, insbesondere von nichtionischen Alkyl- oder Alkylarylpolyethylenoxidethern gegebenenfalls zusammen mit Gallensäure-Salzen wie Na-Cholat, das zusätzlich als Lösungsvermittler dient, ohne die die Cholesterin-Esterase keine Hydrolyse-Aktivität entfalten würde. Als Cholesterin-Esterasen werden dabei bevorzugt Enzyme mikrobiellen Ursprungs eingesetzt.

Die Bestimmung des Serum-HDL-Cholesterins erfolgt nach dem gleichen Prinzip, jedoch müssen dazu zuvor in einem oder mehreren Verfahrensschritten aus dem Serum die anderen cholesterinhaltigen Lipoproteinpartikel (Chylomikronen, VLDL und LDL) abgetrennt werden.

Zur Abtrennung dieser Lipoproteine werden vor allem die Ultrazentrifugation, Elektrophorese oder diverse Präzipitationsmethoden wie Immunpräzipitation, Fällung mit Phosphowolframsäure/$Mg^{2+}$, Dextransulfat/$Mg^{2+}$ oder Heparin/$Mn^{2+}$ oder mit Polyethylenglykol verwendet (s. z. B. Ärztl. Lab. <u>29</u>, 107-114 (1983); Clin. Chem., <u>31</u>, 252-256 (1985)).

Neben der hierfür zusätzlich notwendigen apparativen Ausrüstung erfordern diese Trennverfahren einen zum Teil beträchtlichen zeitlichen und personellen Aufwand, was die routinemäßige HDL-Cholesterin-Bestimmung im klinischen Labor erheblich erschwert und damit auch verteuert.

Wegen des gleichermaßen hohen diagnostischen Stellenwerts der Gesamt- wie auch HDL-Cholesterinbestimmung im Serum ist es daher um so wichtiger, über ein Verfahren und Reagenz zu verfügen, das die Analyse von HDL-Cholesterin und Serum-Gesamtcholesterin in einem Reaktionsansatz ohne die Notwendigkeit einer speziellen Probenvorbereitung ermöglicht.

Ziel der Erfindung ist es daher, die HDL-Cholesterinbestimmung im Serum oder Plasma ohne vorherige Abtrennung von LDL-Cholesterin, VLDL-Cholesterin und Chylomikronen-Cholesterin aus der Probe direkt durchführbar zu machen. Ein weiteres Ziel der Erfindung ist es, ein derartiges Verfahren in Kombination mit der Gesamtcholesterinbestimmung im Serum oder Plasma vorzunehmen.

Dies wird erfindungsgemäß erreicht durch ein Verfahren zur spezifischen Bestimmung von HDL-Cholesterin im Serum oder Plasma durch Inkubation mit einem Cholesterinnachweissystem, enthaltend Cholesterinoxidase und Cholesterinesterase in gepuffertem wäßrigem Medium und Messung eines Produktes der Cholesterinoxidase-Reaktion oder des Sauerstoffverbrauchs, welches dadurch gekennzeichnet ist, daß man das Serum oder Plasma direkt ohne vorherige Abtrennung von anderen cholesterinhaltigen Lipoproteinen in Gegenwart eines Gallensäure- oder Gallensäurederivatsalzes oder von Dioctylsulfosuccincat (Aerosol®OT) inkubiert, dann eine erste Messung durchführt, anschließend ein nichtionisches Polyethylenoxidgruppen enthaltendes Detergenz oder ein sekundäres Alkansulfonat zusetzt, erneut inkubiert und dann eine zweite Messung durchführt und die HDL-Cholesterinmenge aus der Differenz der ersten und der zweiten Messung ermittelt.

Die Erfindung beruht auf der überraschenden Feststellung, daß bestimmte Detergenzien nur das in den Chylomikronen, VLDL und LDL enthaltene Cholesterin der Umsetzung mit Cholesterinesterase und Cholesterinoxidase zugänglich machen, so daß es möglich ist, diese Cholesterinfraktionen in einem ersten Inkubationsschritt mit den genannten Enzymen abreagieren zu lassen und erst dann in einem zweiten Inkubationsschritt durch Zusatz eines nichtionischen, Polyethylenoxidgruppen enthaltenden Detergenzes oder eines sekundären Alkansulfonats das HDL-Cholesterin der Umsetzung zuzuführen. Dies ermöglicht es, in einem einzigen Reaktionsansatz entweder zuerst das in den Chylomikronen, VLDL und LDL enthaltene Cholesterin und danach separat das HDL-Cholesterin zu bestimmen, so daß man sowohl Gesamtcholesterin als auch HDL-Cholesterin in einem einzigen Ansatz erhält oder das Chylomikronen-, VLDL- und LDL-Cholesterin ohne Bestimmung erst abreagieren zu lassen und dann lediglich das HDL-Cholesterin zu messen. Im ersten Fall wird die HDL-Cholesterinmenge aus der Differenz der Messungen nach der ersten Inkubation und der zweiten Inkubation und die Gesamtcholesterinmenge aus der Messung nach der zweiten Inkubation allein ermittelt. Im zweiten Fall, zur alleinigen Bestimmung von HDL-Cholesterin,ist während der ersten Inkubation ein Phenol, Phenolderivat oder ein Anilin sowie Peroxidase zugegen, jedoch kein Aminoantipyrin, MBTH oder MBTH-S. Dadurch reagiert das gemäß Gleichung 2 entstandene $H_2O_2$ ohne Farbbildung ab. Erst bei Durchführung der zweiten Inkubation in Gegenwart von 4AAP, MBTH oder MBTH-S wird Farbe gebildet, die ein direktes Maß für die HDL-Konzentration ist. Bei dieser Verfahrensabwandlung ist zur HDL-Bestimmung demnach nur eine Messung erforderlich.

Vorzugsweise wird daher das erfindungsgemäße Verfahren so durchgeführt, daß man ein Cholesterinnachweissystem verwendet, welches $H_2O_2$ als Produkt der Cholesterinoxidasereaktion 2 durch Farbbildung mißt. Entsprechende chromogene Systeme, die sich für den $H_2O_2$-Nachweis eignen, sind dem Fachmann bekannt. Bevorzugt wird das sogenannte Trindersystem, welches aus Phenol bzw. Phenolderivaten oder substituierten Anilinen, 4-Aminoantipyrin und Peroxidase besteht. Unter den geeigneten Phenolen bzw. Derivaten haben vor allem Phenol, Chlorphenol, Trijod bzw. Tribrom-Hydroxybenzoesäure und Dichlorphenolsulfonsäure Bedeutung erlangt. Anstelle von 4-Aminoantipyrin kann beispielsweise auch 3-Methyl-2-benzothiazolinon-hydrazon (MBTH) und dessen sulfoniertes Derivat (MBTHS) eingesetzt werden, zweckmäßig in Kombination mit substituierten Anilinen wie Dimethylanilin, Dimethylaminobenzoesäure, **N**-Ethyl-**N**-β-sulfoethyl-m-toluidin oder **N**-Ethyl-N-β-Hydroxyethyl-m-toluidin, wobei sich die letzten beiden Substanzen auch in Kombination mit 4-Aminoantipyrin bewährt haben.

Als Cholesterinesterase kommen mikrobielle Cholesterinesterase, z. B. aus Candida cylindracea oder das Pankreasenzym, welches bevorzugt wird, in betracht.

Als Gallensäure- bzw. Gallensäurederivat sind die Alkalimetall- bzw. Magnesiumsalze von Cholsäure, Desoxycholsäure, Lithocholsäure, Taurocholsäure, Glykocholsäure, Desoxyglykocholsäure und Desoxytaurocholsäure und Choleinsäuren zu erwähnen. Bevorzugt wird Natriumcholat. Alternativ kann auch Na-Dioctylsulfosuccinat (Aerosol®OT) verwendet werden. Es wurde gefunden, daß diese Detergenzien nur die lipidreichen und mit einem relativ geringen Proteinanteil ausgestatteten Lipoproteine Chylomikronen, VLDL und LDL aufschließen und das in ihnen enthaltene Cholesterin für die enzymatische Umsetzung zugänglich machen.

Das Cholesterin der HDL-Fraktion wird durch nichtionische, Polyethylenoxidgruppen enthaltende Detergenzien wie die nichtionischen Alkyl- oder Alkylaryl-polyethylenoxidether, z. B. n-Dodecylpolyethylenglykolether (Thesit®), iso-Tridekanolpolyethylenoxidether (Genapol®x-80) iso-Octylphenolpolyethylenoxidether (Triton®X-100) der enzymatischen Bestimmung zugänglich gemacht. Auch anionische sekundäre Alkansulfonate, sie beispielsweise unter der Bezeichnung Hostapur®AT im Handel sind, können verwendet werden. Vorzugsweise wird diese Gruppe von HDL aufschließenden Detergenzien zusammen mit Methanol, Ethanol, n-Propanol oder Isopropanol eingesetzt. Bevorzugt wird Propanol verwendet. Vorzugsweise beträgt die Alkoholmenge 0,05 bis 2 %. Diese und alle anderen Konzentrationsangaben beziehen sich auf die Endkonzentration im Reaktionsgemisch.

Das Gallensäuresalz bzw. Gallensäurederivatsalz wird vorzugsweise bis zu einer Konzentration zwischen 0,2 und 20 mmol/l zugesetzt. Diese Mengen können aber auch je nach der zu untersuchenden Probe unter- oder überschritten werden. Für das Dioctylsulfosuccinat gilt das gleiche.

Hinsichtlich des zweiten Detergenz, also des nichtionische Polyethylenoxidgruppen enthaltenden Detergenz bzw. des sekundären Alkansulfonats liegt die bevorzugte Konzentration zwischen 0,02 und 2%. Je nach Probe können aber auch diese Konzentrationen über- bzw. unterschritten werden.

Im übrigen wird bei der Umsetzung wie bei der bekannten Cholesterinbestimmung gearbeitet hinsichtlich pH-Wert, geeigneten Puffersubstanzen und Mengen der einzusetzenden Enzyme.

Ein weiterer Gegenstand der Erfindung ist eine Reagenzienkombination zur spezifischen Bestimmung von HDL-Cholesterin im Serum oder Plasma, enthaltend Cholesterinoxidase, Puffer und Cholesterinesterase, die dadurch gekennzeichnet ist, daß sie aus wenigstens 2 nicht vermischten Einzelreagenzien besteht, deren erstes, bezogen auf die Endkonzentration im Test,
0,1 bis 10 U/ml Cholesterinesterase,
0,05 bis 10 U/ml Cholesterinoxidase,
20 bis 500 mmol/l Puffersubstanz pH 6,0 bis 8,0,
0,2 bis 20 mmol/l Gallensäure oder Gallensäurederivate oder Dioctylsulfosuccinat, und
deren zweites, bezogen auf die Endkonzentration im Test

0,02 bis 2% nichtionisches Polyethylenoxidgruppen enthaltendes Detergenz oder sekundäres Alkansulfonat sowie gegebenenfalls
0,05 bis 2% Alkohol mit bis zu 3 C-Atomen enthält.

Gemäß einer ersten Ausführungsform enthält die erfindungsgemäße Reagenzienkombination im ersten Einzelreagenz zusätzlich 0,05 bis 20 U/ml Peroxidase, 0,2 bis 20 mmol/l eines Phenols oder Anilins und gegebenenfalls 0,02 bis 5% Polyethylenglykol und das zweite Einzelreagenz 0,05 bis 2% n-Propanol.

Eine bevorzugte Reagenzienkombination gemäß der Erfindung enthält im ersten Einzelreagenz
0,2 bis 2 U/ml Cholesterinesterase,
0,05 bis 0,5 U/ml Cholesterinoxidase,
1 bis 5 U/ml Peroxidase,
50 bis 200 mmol/l Kaliumphosphatpuffer pH 6,5 bis 7,0,
1,4 bis 10 mmol/l Phenol oder Phenolderivat,
1 bis 5 mmol/l Gallensäure- oder Gallensäurederivatsalz oder Dioctylsulfosuccinat, gegebenenfalls getrennt davon 0,2 bis 2 mmol/l 4-Aminoantipyrin und getrennt von den anderen Bestandteilen, ausgenommen gegebenenfalls 4-Aminoantipyrin, im zweiten Einzelreagenz
0,5 bis 5,0% nichtionisches, Polyethylenoxidgruppen enthaltendes Detergenz oder sekundäres Alkansulfonat und gegebenenfalls
0,1 bis 0,5% n-Propanol und
0,5 bis 5,0% Polyäthylenglykol.

In einer weiteren Ausführungsform liegen die genannten Reaktionskomponenten auf oder in einem Trägermaterial imprägniert vor. Als Trägermaterial kann ein saugfähiges oder quellfähiges oder filmbildendes Trägermaterial, z.B. ein für Teststreifen bekanntes Trägermaterial wie Papier und ähnliche Vliesmaterialien, beispielsweise Teebeutelpapier, verwendet werden. Dabei können die Reaktionskomponenten auf mehrere in Kontakt stehende Träger verteilt sein.

Durch die Erfindung wird erreicht, daß das im HDL enthaltende Cholesterin direkt ohne vorherige Abtrennung der anderen, Cholesterin enthaltenden Lipoproteinfraktionen, bestimmt werden kann. Dies bedeutet eine wesentliche Erleichterung und Vereinfachung der Bestimmungsmethode.

Das folgende Beispiel erläutert die Erfindung weiter.

Dadei werden nachstehende Abkürzungen verwendet:

| | | |
|---|---|---|
| CHOD | = | Cholesterinoxidase |
| CHOL-Esterase | = | Cholesterinesterase |
| POD | = | Peroxidase |
| 4-AAP | = | 4-Aminoantipyrin |
| PEG 6000 | = | Polyäthylenglykol 6000 |
| TRA | = | Triäthanolamin |

| Beispiel 1 | | | |
|---|---|---|---|
| a) Grundreagenz | K-Phosphat 0,1 mol/1 pH 6,7 | | |
| | 3mM Na-Cholat | | |
| | 0,8 mM 4-AAP | CHOD 1,2 U/ml | |
| | 1,4 mM Phenol | CHOL. Esterase 0,5 U/ml | |
| | 0,1% PEG 6000 | | |
| | POD 3 U/ml | | |
| b) Nachstartreagenz | TRA-Puffer 0,05 m pH 7,1 | | |
| | 10% Thesit | | |
| | 10% Propanol | | |
| c) Durchführung des Tests | Wellenlänge | 546 nm | |
| | Temperatur | 25°C | |
| | Messung bei 25°C gegen Reagenzleerwert. | | |
| | In Küvetten mit einer Schichtdicke von 10 mm pipettieren: | | |
| | Probe | Reagenzleerwert (RLW) | |
| Grundreagenz | 2,00 ml | 2,00 ml | |
| Probe | 0,02 ml | | |
| Wasser | – | 0,02 ml | |
| 10 (–30) Minuten inkubieren, anschließend $E_1$ (Probe) gegen $E_1$ (RLW) messen (= $E_1$) | | | |
| Nachstartreagenz | 0,02 ml | 0,02 ml | |
| mindestens weitere 10 Minuten inkubieren, anschließend $E_2$ (Probe) gegen $E_2$ (RLW) messen (= $E_2$) | | | |

d) Berechnung:

Die Berechnung erfolgt durch Mitführen eines Serum-Standards mit bekannter Gesamt- und HDL-Cholesterin-Konzentration. Der Serum-Standard ist dabei im Testansatz wie Probe einzusetzen.

$$c\left[\text{Gesamtcholesterin}\right] \text{(mg/dl} = c_{\text{Standard}} \times \frac{\Delta E_2\text{Probe}}{\Delta E_2\text{Standard}}$$

$$c\left[\text{HDL-Cholesterin}\right] \text{(mg/dl)} = c_{\text{Standard}} \times \frac{(\Delta E_2 - \Delta E_1)\ \text{Probe}}{(\Delta E_2 - \Delta E_1)\ \text{Standard}}$$

Der zeitliche Verlauf der entsprechend dem Testansatz gemäß Beispiel erfolgenden stufenweisen Farbbildung ist in Fig. 1 der Zeichnung wiedergegeben.

| Beispiel 2 | | | |
|---|---|---|---|
| a) Grundreagenz | Wie in Beispiel 1, jedoch ohne 4-AAP | | |
| b) Nachstartreagenz | Wie in Beispiel 1, jedoch zusätzlich 80 mM 4-AAP | | |
| c) Durchführung des Tests | Wellenlänge | 546 nm | |
| | Temperatur | 25°C | |
| | Messung bei 25°C gegen Reagenzleerwert. | | |
| | In Küvetten mit einer Schichtdicke von 10 mm pipettieren: | | |
| | Probe | Reagenzleerwert (RLW) | |
| Grundreagenz | 2,00 ml | 2,00 ml | |
| Probe | 0,02 ml | | |
| Wasser | – | 0,02 ml | |
| 10 (–30) Minuten inkubieren, | | | |
| Nachstartreagenz | 0,02 ml | 0,02 ml | |
| mindestens weitere 10 Minuten inkubieren, anschließend E (Probe) gegen E (RLW) messen (= ΔE). | | | |

d) Berechnung:

Die Berechnung erfolgt durch Mitführen eines Serum-Standards mit bekannter Gesamt- und HDL-Cholesterinkonzentration. Der Serum-Standard ist dabei im Testansatz wie Probe einzusetzen.

$$c\,[\text{HDL-Cholesterin}]\ (\text{mg/dl}) = c_{\text{Standard}} \times \frac{\Delta E\ \text{Probe}}{\Delta E\ \text{Standard}}$$

Die Gegenüberstellung der HDL-Cholesterinbestimmung gemäß Beispiel 2 in verschiedenen Humanseren zwischen dem erfindungsgemäßen Verfahren und der auf der Phosphorwolframsäure (PWS) /Mg-Sulfat-Fällung beruhenden Methode ist in Fig. 2 dargestellt. Der Methodenvergleich ergibt eine Regression von Y =-0,907 + 1,019 X mit einem Korrelationskoeffizienten von 0,814.

Identische Resultate für HDL-Cholesterin werden erhalten, wenn man gemäß Beispiel 1 arbeitet.

**Patentansprüche**

1. Verfahren zur spezifischen Bestimmung von HDL-Cholesterin im Serum oder Plasma durch Inkubation mit einem Cholesterinnachweissystem, enthaltend Cholesterinoxidase und Cholesterinesterase in gepuffertem wäßrigem Medium und Messung eines Produktes der Cholesterinoxidase-Reaktion oder des Sauerstoffverbrauchs, dadurch gekennzeichnet, daß man das Serum oder Plasma direkt ohne vorherige Abtrennung von anderen cholesterinhaltigen Lipoproteinen in Gegenwart eines Gallensäure- oder Gallensäurederivatsalzes oder von Dioctylsulfosuccinat inkubiert, dann eine erste Messung durchführt, anschließend ein nichtionisches Polyethylenoxidgruppen enthaltendes Detergenz oder ein sekundäres Alkansulfonat zusetzt, erneut inkubiert und dann eine zweite Messung durchführt und die HDL-Cholesterinmenge aus der Differenz der ersten und der zweiten Messung ermittelt.

2. Verfahren nach Anspruch 1, **dadurch gekennzeichnet**, daß man ein Cholesterinnachweissystem verwendet, welches mit $H_2O_2$ als Produkt der Cholesterinoxidase-Reaktion eine Farbbildung bewirkt.

3. Verfahren nach Anspruch 2, **dadurch gekennzeichnet**, daß man zur Farbbildung ein chromogenes System aus einem Phenol oder einem Anilin, 4-Aminoantipyrin und Peroxidase einsetzt.

4. Abwandlung des Verfahrens gemäß Anspruch 1, **dadurch gekennzeichnet**, daß man eine erste Inkubation in Gegenwart von

a) Gallensäure- oder Gallensäurederivatsalzes oder von Dioctylsulfosuccinat,

b) Phenol oder Anilin und

c) von Peroxidase durchführt, anschließend ein nichtionisches, Polyethylenoxidgruppen enthaltendes Detergenz oder ein sekundäres Alkansulfonat und 4-Aminoantipyrin zusetzt, erneut inkubiert und dann die gebildete Färbung mißt.

5. Verfahren nach einem der vorhergehenden Ansprüche, **dadurch gekennzeichnet**, daß man Cholesterinesterase aus Pankreas verwendet.

6. Verfahren nach einem der vorhergehenden Ansprüche, **dadurch gekennzeichnet**, daß man das Gallensäure- oder Gallensäurederivatsalz oder Dioctylsulfosuccinat bis zu einer Konzentration zwischen 0,2 und 20 mmol/l zusetzt.

7. Verfahren nach einem der vorhergehenden Ansprüche, **dadurch gekennzeichnet**, daß man das nichtionische Polyethylenoxidgruppen enthaltende Detergenz oder das sekundäre Alkansulfonat bis zu einer Konzentration zwischen 0,02 bis 2 % zusetzt.

8. Verfahren nach einem der vorhergehenden Ansprüche, **dadurch gekennzeichnet**, daß man die Umsetzung zwischen pH 6,0 und 8,0 durchführt.

9. Verfahren nach einem der vorhergehenden Ansprüche, **dadurch gekennzeichnet**, daß man dem Reaktionsmedium 0,05 bis 2 % Alkohol mit 1 bis 3 C-Atomen zusetzt.

10. Reagenzienkombination zur spezifischen Bestimmung von HDL-Cholesterin im Serum oder Plasma, enthaltend Cholesterinoxidase, Puffer und Cholesterinesterase, dadurch gekennzeichnet, daß sie aus wenigstens 2 nicht vermischten Einzelreagenzien besteht, deren erstes, bezogen auf die Endkonzentration im Test,
0,1 bis 10 U/ml Cholesterinesterase,
0,05 bis 10 U/ml Cholesterinoxidase,
20 bis 500 mmol/l Puffersubstanz pH 6,0 bis 8,0,
0,2 bis 20 mmol/l Gallensäure oder Gallensäurederivate oder Dioctylsulfosuccinat,
und deren zweites, bezogen auf die Endkonzentration im Test
0,02 bis 2% nichtionisches Polyethylenoxidgruppen enthaltendes Detergenz oder sekundäres Alkansulfonat, sowie gegebenenfalls
0,05 bis 2% Alkohol mit bis zu 3 C-Atomen enthält.

11. Reagenzienkombination nach Anspruch 10, dadurch gekennzeichnet, daß das erste Einzelreagenz zusätzlich
0,05 bis 20 U/ml Peroxidase,
0,2 bis 20 mmol/l eines Phenols, Phenolderivats oder Anilins und gegebenenfalls
0,02 bis 5% Polyethylenglykol und das zweite Einzelreagenz gegebenenfalls
0,05 bis 2% n-Propanol enthält.

12. Reagenzienkombination nach Anspruch 11, dadurch gekennzeichnet, daß das erste Einzelreagenz
0,05 bis 0,5 U/ml Cholesterinoxidase,
0,2 bis 2 U/ml Peroxidase,
1 bis 5 U/ml Cholesterinesterase,
50 bis 200 mmol/l Kaliumphosphatpuffer pH 6,5 bis 7,0,
1,4 bis 10 mmol/l Phenol oder Phenolderivat,
1 bis 5 mmol/l Gallensäure- oder Gallensäurederivatsalz oder Dioctylsulfosuccinat, gegebenenfalls
0,1 bis 0,5% Polyäthylenglykol und das zweite Einzelreagenz
0,05 bis 0,5% nichtionisches Polyethylenoxidgruppen enthaltendes Detergenz oder sekundäres Alkansulfonat und gegebenenfalls
0,1 bis 0,5% n-Propanol enthält und eines der Einzelreagenzien gegebenenfalls
0,2 bis 2 mmol/l 4-Aminoantipyrin enthalten kann.

**Claims**

1. Process for the specific determination of HDL cholesterol in serum or plasma by incubation with a cholesterol detection system, containing cholesterol oxidase and cholesterol esterase in buffered aqueous medium, and measurement of a product of the cholesterol oxidase reaction or of the oxygen consumption, characterised in that one incubates the serum or plasma directly, without previous separation from other cholesterol-containing lipoproteins, in the presence of a bile acid or bile acid derivative salt or of dioctylsulphosuccinate, then carries out a first measurement, subsequently adds thereto a non-ionic, polyethylene oxide group-containing detergent or a secondary alkane sulphonate, again incubates and then carries out a second measurement and determines the amount of HDL cholesterol from the difference of the first and second measurement.

2. Process according to claim 1, characterised in that one uses a cholesterol detection system which, with $H_2O_2$ as the product of the cholesterol oxidase reaction, brings about a colour formation.

3. Process according to claim 2, characterised in that, for the colour formation, one uses a chromogenic system of a phenol or an aniline, 4-aminoantipyrine and peroxidase.

4. Modification of the process according to claim 1, characterised in that one carries out a first incubation in the presence of
a) bile acid or bile acid derivative salt or of dioctylsulphosuccinate,
b) phenol or aniline or
c) of peroxidase, subsequently adds thereto a non-ionic, polyethylene oxide group-containing detergent or a secondary alkane sulphonate and 4-aminoantipyrine again incubates and then measures the colour formed.

5. Process according to one of the preceding claims, characterised in that one uses cholesterol esterase from the pancreas.

6. Process according to one of the preceding claims, characterised in that one adds the bile acid or bile acid derivative salt or dioctylsulphosuccinate up to a concentration between 0.2 and 20 mmole/l.

7. Process according to one of the preceding claims, characterised in that one adds the non-ionic polyethylene oxide group-containing detergent or the secondary alkane sulphonate up to a concentration between 0.02 and 2%.

8. Process according to one of the preceding claims, characterised in that one carries out the reaction between pH 6.0 and 8.0.

9. Process according to one of the preceding claims, characterised in that one adds to the reaction medium 0.05 to 2% of alcohol with 1 to 3 C-atoms.

10. Reagent combination for the specific determination of HDL cholesterol in serum or plasma, containing cholesterol oxidase, buffer and cholesterol esterase, characterised in that it consists of at least 2 non-mixed individual reagents, the first of which, referred to the end concentration in the test, contains
0.1 to 10 U/ml. cholesterol esterase,
0.05 to 10 U/ml. cholesterol oxidase,
20 to 500 mmole/l. buffer substance pH 6.0 to 8.0
0.2 to 20 mmole/l. bile acid or bile acid derivative or dioctylsulphosuccinate,
and the second of which, referred to the end concentration of the test, contains
0.02 to 2% non-ionic polyethylene oxide group-containing detergent or secondary alkane sulphonate, as well as possibly 0.05 to 2% alcohol with up to 3 C-atoms.

11. Reagent combination according to claim 10, characterised in that the first individual reagent additionally contains 0.05 to 20 U/ml. peroxidase,
0.2 to 20 mmol/l. of a phenol, phenol derivative or aniline and possibly 0.02 to 5% polyethylene glycol and the second individual reagent possibly contains 0.05 to 2% n-propanol.

12. Reagent combination according to claim 11, characterised in that the first individual reagent contains
0.05 to 0.5 U/ml. cholesterol oxidase,
0.2 to 2 U/ml. peroxidase,
1 to 5 U/ml. cholesterol esterase,
50 to 200 mmole/l. potassium phosphate buffer pH 6.5 to 7.0,
1.4 to 10 mmole/l. phenol or phenol derivative,
1 to 5 mmole/l. bile acid or bile acid derivative salt or dioctylsulphosuccinate, possibly
0.1 to 0.5% polyethylene glycol
and the second individual reagent contains
0.05 to 0.5% non-ionic polyethylene oxide group-containing detergent or secondary alkane sulphonate and possibly
0.1 to 0.5% n-propanol and/or polyethylene glycol and one of the individual reagents can possibly contain
0.2 to 2 mmol/l. 4-aminoantipyrine.

**Revendications**

1. Procédé pour la détermination spécifique de HDL-cholestérol dans le sérum ou le plasma par incubation avec un système de mise en évidence du cholestérol, contenant de la cholestérol-oxydase et de la cholestérol-estérase en milieu aqueux tamponné et mesure d'un produit de la réaction de la cholestérol-oxydase ou de la consommation d'oxygène, caractérisé en ce qu'on incube le sérum ou le plasma directement, sans séparation préalable des autres lipoprotéines contenant du cholestérol, en présence d'un sel d'acide biliaire ou d'un sel de dérivé d'acide biliaire ou de sulfosuccinate de dioctyle, puis on effectue une première mesure, on ajoute ensuite un détergent non ionique contenant des groupes polyéthylène-oxyde ou un alcanesulfonate secondaire, on incube de nouveau puis on effectue une deuxième mesure et on détermine la quantité de HDL-cholestérol à partir de la différence des première et deuxième mesures.

2. Procédé selon la revendication 1, caractérisé en ce qu'on utilise un système de mise en évidence du cholestérol qui produit une formation de couleur avec $H_2O_2$ en tant que produit de la réaction de la cholestérol-oxydase.

3. Procédé selon la revendication 2, caractérisé en ce qu'on met en œuvre, pour la formation de couleur, un système chromogène constitué d'un phénol ou d'une aniline, de 4-aminoantipyrine et de peroxydase.

4. Modification du procédé selon la revendication 1, caractérisée en ce qu'on effectue une première incubation en présence
a) de sel d'acide biliaire ou de sel de dérivé d'acide biliaire ou de sulfosuccinate de dioctyle,
b) de phénol ou d'aniline, et
c) de peroxydase,
on ajoute ensuite un détergent non ionique, contenant des groupes polyéthylène-oxyde ou un alcanesulfonate secondaire et de la 4-aminoantipyrine, on incube de nouveau puis on mesure la coloration formée.

5. Procédé selon l'une des revendications précédentes, caractérisé en ce que l'on utilise de la cholestérol-estérase du pancréas.

6. Procédé selon l'une des revendications précédentes, caractérisé en ce qu'on ajoute le sel d'acide gallique ou de dérivé d'acide gallique ou le sulfosuccinate de dioctyle jusqu'à une concentration comprise entre 0,2 et 20 mmoles/l.

7. Procédé selon l'une des revendications précédentes, caractérisé en ce qu'on ajoute le détergent nonionique contenant des groupes polyéthylène-oxyde ou l'alcanesulfonate secondaire jusqu'à une concentration comprise entre 0,02 jusqu'à 2%.

8. Procédé selon l'une des revendications précédentes, caractérisé en ce qu'on effectue la réaction entre pH 6,0 et 8,0.

9. Procédé selon l'une des revendications précédentes, caractérisé en ce qu'on ajoute au milieu réactionnel 0,05 à 2% d'alcool comprenant 1 à 3 atomes de C.

10. Combinaison de réactifs pour la détermination spécifique de HDL-cholestérol dans le sérum ou le plasma, contenant de la cholestérol-oxydase, du tampon et de la cholestérol-estérase, caractérisée en ce qu'elle est constituée d'au moins 2 réactifs individuels non mélangés, dont le premier contient, par rapport à la concentration finale dans l'essai,
0,1 à 10 U/ml de cholestérol-estérase,
0,05 à 10 U/ml de cholestérol-oxydase,
20 à 500 mmoles/l de substance tampon pH 6,0 à 8,0,
0,2 à 20 mmoles/l d'acide gallique ou de dérivés d'acide gallique ou de sulfosuccinate de dioctyle, et dont le second contient, par rapport à la concentration finale dans l'essai
0,02 à 2% de détergent non ionique contenant des groupes polyéthylène-oxyde ou de l'alcanesulfonate secondaire, ainsi qu'éventuellement
0,05 à 2% d'alcool ayant jusqu'à 3 atomes de C.

11. Combinaison de réactifs selon la revendication 10, caractérisée en ce que le premier réactif individuel contient en outre
0,05 à 20 U/ml de peroxydase,
0,2 à 20 mmoles/l d'un phénol, d'un dérivé de phénol ou d'une aniline, et éventuellement
0,02 à 5% de polyéthylène-glycol et le second réactif individuel contient éventuellement
0,05 à 2% de n-propanol.

12. Combinaison de réactifs selon la revendication 11, caractérisée en ce que le premier réactif individuel contient
0,05 à 0,5 U/ml de cholestérol-oxydase,
0,2 à 2 U/ml de peroxydase,
1 à 5 U/ml de cholestérol-estérase,
50 à 200 mmoles/l de tampon phosphate de potassium pH 6,5 à 7,0,
1,4 à 10 mmoles/l de phénol ou de dérivé de phénol,
1 à 5 mmoles/l de sel d'acide gallique ou de sel de dérivé d'acide gallique ou de sulfosuccinate de dioctyle, éventuellement
0,1 à 0,5% de polyéthylène-glycol
et en ce que le second réactif individuel contient
0,05 à 0,5% de détergent non ionique contenant des groupes polyéthylène-oxyde ou de l'alcanesulfonate secondaire et éventuellement
0,1 à 0,5% de n-propanol
et en ce que l'un des réactifs individuels peut éventuellement contenir
0,2 à 2 mmoles/l de 4-aminoantipyrine.

FIG.1

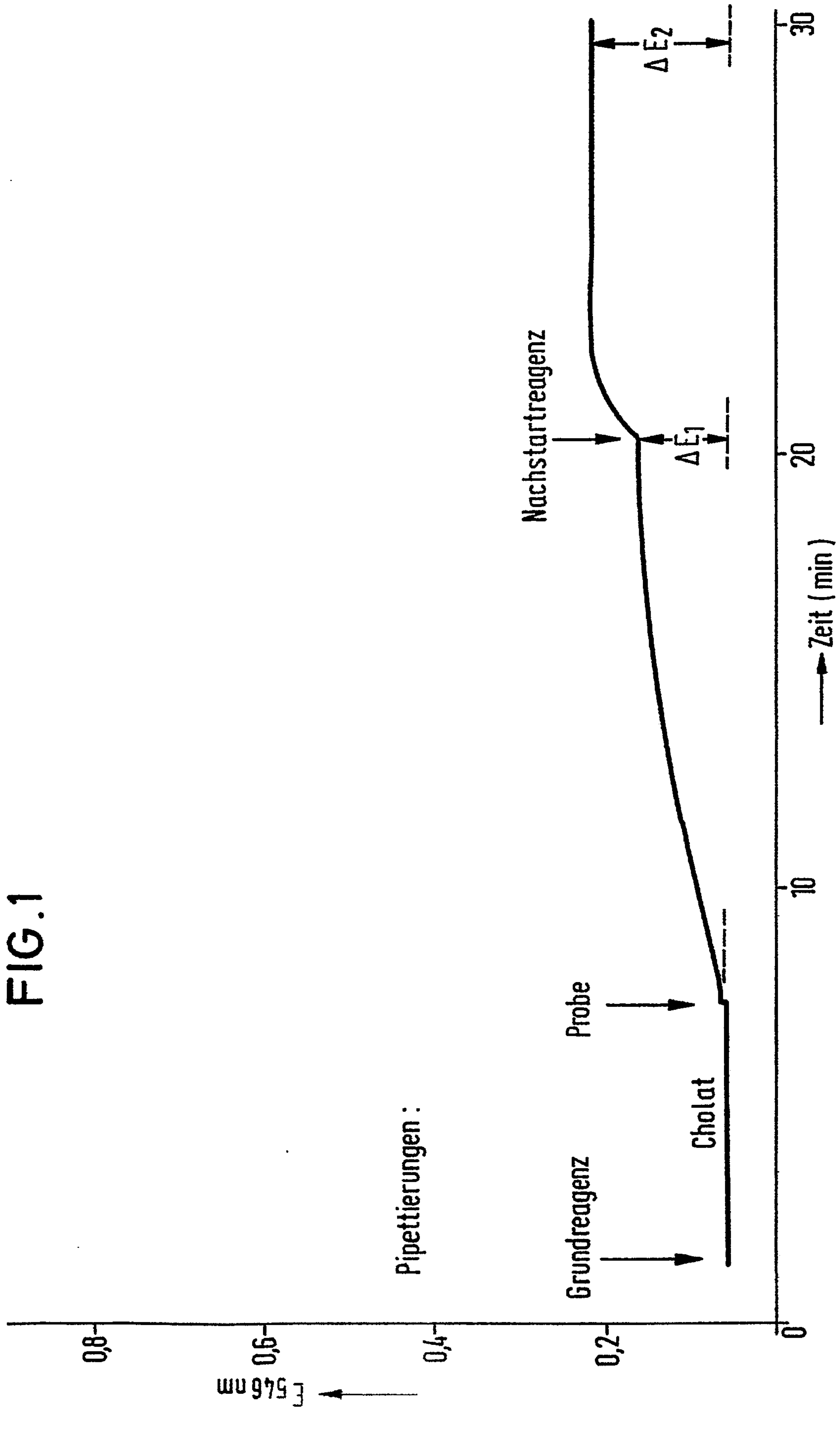
Pipettierungen :
Grundreagenz
Cholat
Probe
Nachstartreagenz
Δ E1
Δ E2
E 546 nm
0,8
0,6
0,4
0,2
0
10
20
30
Zeit (min)

FIG.2

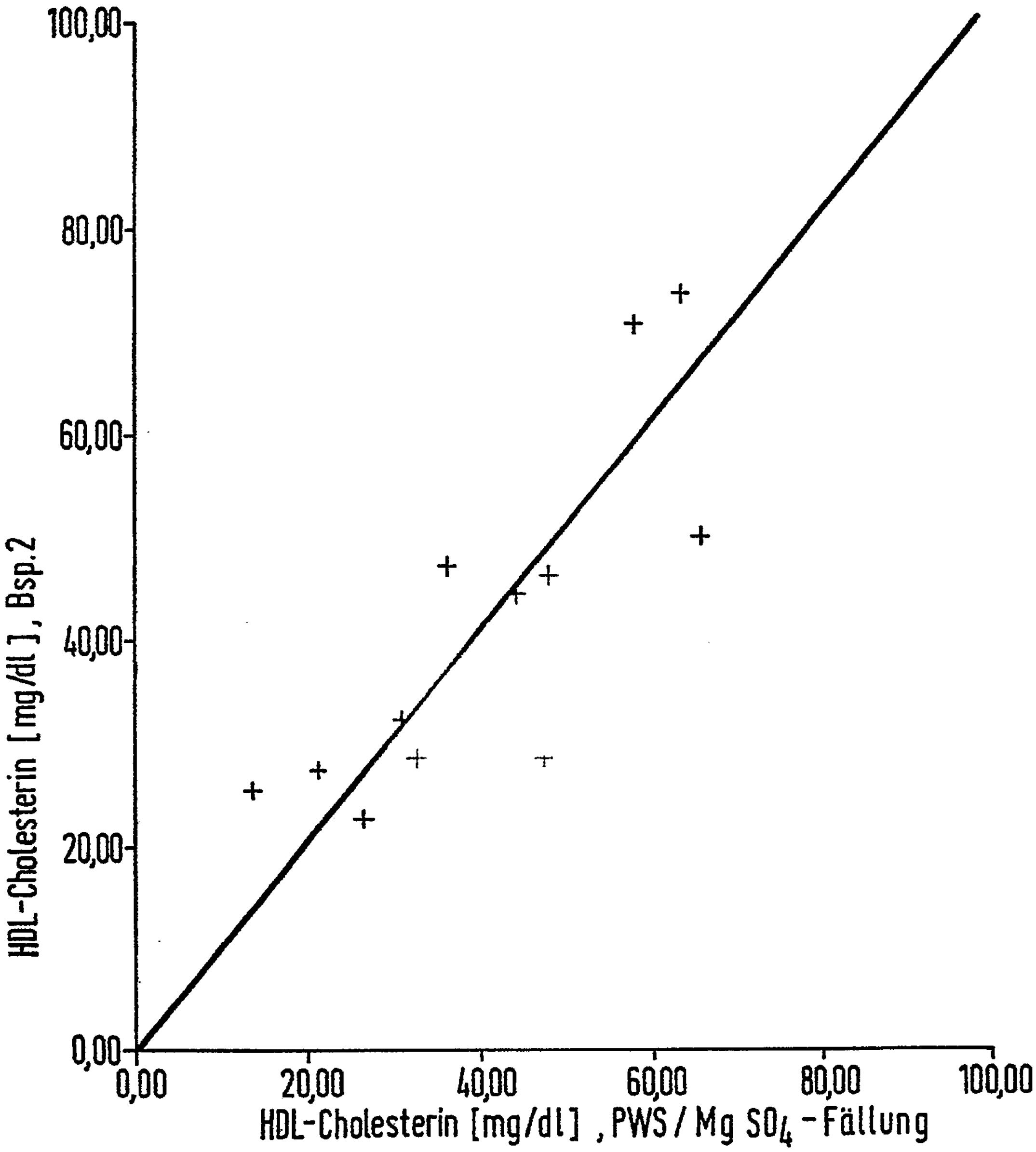
100,00
80,00
60,00
40,00
20,00
0,00
0,00
20,00
40,00
60,00
80,00
100,00
HDL-Cholesterin [mg/dl], Bsp.2
HDL-Cholesterin [mg/dl] , PWS / Mg $SO_4$ - Fällung